# EUROPEAN PATENT APPLICATION

(11) **EP 2 514 472 A2**
(43) Date of publication of application: **24.10.2012**
(21) Application number: 12163856.3
(22) Date of filing: 12.04.2012
(51) Int. Cl.: A61M 16/08, F16T 1/20

(54) **Automatic water removal device and method**

(30) Priority: 21.04.2011 US 201113091673
(71) Applicant: Teleflex Medical Incorporated, Durham, NC 27709 (US)
(72) Inventor: Dwyer, Daniel Patrick, Raleigh, North Carolina 27613 (US); DeVille, Jarred, Wake Forest, North Carolina 27587 (US)
(74) Representative: Nguyen Van Yen, Christian

(57) **Abstract**

A device (100) and method is provided for automatically removing water or accumulated moisture from a breathing circuit. The device includes a housing (101) with breathing circuit flow entry and exit ports (130,132) and a water drainage port (120). A buoyant body (112) in the housing can mate with a float valve seat (121) to seal the drainage port, and is moveable to open the drainage port when sufficient liquid (L) accumulates in the interior space. The buoyant body displaces a substantial portion of the housing inner compressible volume. Water is automatically removed by a water vapor permeable container (102) coupled to the housing receiving flow from the drainage port, or the device is coupled to a source of suction, further including at least one bleed port (360) fluidly coupling the source of suction to the surroundings, acting with a valve element (388) to equalize any pressure differential between the housing interior space and suction connection, when no water is accumulated in the device.

## Description

### FIELD OF THE INVENTION

The present invention relates generally to a medical device. More particularly, the present invention is related to a water removal or dissipation device for placement with a breathing circuit.

### BACKGROUND

A breathing circuit delivers medical gas to a patient under pressure in a prescribed volume and breathing rate. The medical gas is often humidified by a humidifier located at or near the ventilator or respirator. The optimum respiratory circuit delivers 100% RH medical gases @ 35 to 39 degrees C to the patient while reducing the amount of humidity and subsequent condensate delivered back to the ventilator through the expiratory limb. Therefore, the humidified gas has to travel through all or most of the tubing and has time to cool. Cooling of the gas leads to rainout or condensation in the breathing tube and collection of water within the breathing circuit.

Several solutions to the problem of rainout have been developed. One such solution is a heating wire provided along the length of the tube. The wire may be provided within the interior of the tubing or alternatively may be embedded along the interior of the tubing. The wire heats the humidified gas traveling through the tubing to prevent the gas from cooling, thus preventing the problem of water condensing out of the gas traveling through the breathing circuit. However, the manufacture of such heated wire respiratory circuits can be time consuming and costly.

Another such solution, which eliminates the heated wire, is to provide a water collection device somewhere within the breathing circuit. A water collection apparatus is typically placed in the expiratory limb of the respiratory circuit in front of the ventilator or respirator to collect and manually remove excessive condensation prior to the gases entering the ventilator or respirator. It is known that excessive condensate entering a ventilator or respirator from the expiratory limb of a respiratory circuit can harm the device.

Most frequently, the water collection device is designed to trap the condensed water vapor in a removable container, also commonly referred to as a "water trap." An example of such a water trap is shown in U.S. patent no. 4,867,153, which discloses a water removal device having, in one embodiment, a float ball valve within a water trap container that opens a lower drainage opening when sufficient water is accumulated inside the trap container. The drainage opening is further connected to an evacuation tube that can be connected to suction or vacuum (negative gauge) pressure. However this water trap and drainage system is not automatic, and requires a user to monitor any accumulation of water and thereafter manually activate a vacuum valve to achieve complete water removal from the device. Furthermore, the apparatus in U.S. patent no. 4,867,153 provides a relatively small volumetric element or structure inside the compressible volume defined by the water trap container, such that the water trap itself constitutes a significant addition to the overall compressible volume within the breathing circuit, increasing the compliance of the system which is detrimental to the patient.

Another type of device for removal or dissipation of water from a breathing circuit is provided in commonly owned pending U.S. patent application No. 12/539,088, published as U.S. Patent Publication No. US 2010/0012127, assigned to Teleflex Medical Incorporated. In said application, a variety of embodiments are disclosed, including a device having a housing or container with an outer cover structure defining an enclosed volume defining an inner flow space for collecting accumulated water or moisture from a breathing circuit flow, wherein the walls of the housing or cover structure are made at least in part from a water vapor breathable medium, such that water vapor or moisture can collect, condense, and then permeate from the inner flow space through the outer cover structure out of device, thereby automatically removing or dissipating water accumulated in the device from the breathing circuit. However this device may not be able to maintain a completely closed system if leaks form in the water vapor breathable medium or outer cover structure or "skin" surrounding the inner flow space, potentially contaminating the breathing circuit flow. A further drawback is that when aerosol treatments are used in the breathing circuit flow, the matter from such treatments may accumulate on the water vapor breathable medium thereby reducing the effectiveness of the device. Yet another problem presented by such a device is that the housing or container defining the inner flow space defines an additional compressible volume that increases the overall compressible volume and compliance of the system to which the device is coupled, potentially rendering breathing more difficult for the patient. It is therefore desirable to have a device that minimizes such additional compressible volume, is unaffected by the use of aerosol treatments, and is less susceptible to leaks to as to keep the breathing circuit flow closed and uncontaminated from the surroundings.

### SUMMARY

The foregoing problems are solved, and needs are met, to a great extent, by the present invention, wherein an apparatus and method is provided that in different embodiments provides for improved and automatic water removal or dissipation of water or moisture than can accumulate in a breathing circuit. The device includes a housing with breathing circuit flow entry and exit ports and a water drainage port. A buoyant body in the housing can mate with a float valve seat to seal the drainage port, and is moveable to open the drainage port when sufficient liquid accumulates in the interior space. The buoyant body displaces a substantial portion of the inner compressible volume of the housing, which, in one preferred embodiment, can be at least half of said volume. The device includes in one embodiment a flow chamber or container structure having walls made of a water vapor breathable medium, which structure at least partially bounds an inner flow space defined by the device thereby providing an extended area and dwell time for moisture in humidified gases to travel through said flow space and come into contact with the structure walls to permeate through the water vapor breathable medium, and thereby effectively be removed from the breathing circuit to which the device is coupled. Water is automatically removed through such a container coupled to the housing receiving flow from the drainage port, the container being made in part from the water vapor permeable medium. In another embodiment, the housing of the device is coupled to a source of suction, further including at least one bleed port fluidly coupling the source of suction to the surroundings, acting together with a valve element to equalize any pressure differential between the housing interior space and suction connection, when no water is accumulated in the device, to enable the buoyant body to effectively operate as a float valve mechanism. In both embodiments, the housing and buoyant body float valve mechanism therein act as both a "water seal" closing the breathing circuit flow from the surroundings, and as a means to provide an effective and substantially lower compressible volume within the overall breathing circuit system to which the device is attached, while simultaneously providing an automatic means of removing or dissipating a large quantity of water or moisture from the system.

In one embodiment of the present invention, a device for automatically removing water or accumulated moisture from a breathing circuit includes a housing defining an entry port for receiving flow from a breathing circuit, an exit port for transmitting flow to a breathing circuit, a drainage port disposed on a lower end portion of the housing, and a float valve seat disposed about said drainage port. The housing further defines a first interior space fluidly coupled to all said ports. A buoyant float body is disposed in the first interior space defining a lower end configured to mate with the float valve seat to seal said drainage port. The buoyant float body is moveable upwards within the interior space when a sufficient amount of liquid accumulates in the interior space to separate the lower end of said buoyant float body from the float valve seat and permit liquid to flow through the drainage port. The buoyant float body defines a volume which displaces at least half of a volume defined by the first interior space defined by the housing exclusive of any volume spanned by the entry and exit ports. And a container is coupled to the housing for receiving flow exiting from the drainage port. The container defines a second interior space for collection of water or accumulated moisture, the second interior space being closed to the surroundings outside the device The container further includes a wall bounding said second interior space, said wall made at least in part from a material permeable to water vapor and impermeable to liquid water.

In another embodiment, a device for automatically removing water or accumulated moisture from a breathing circuit includes a housing defining an entry port for receiving flow from a breathing circuit, an exit port for transmitting flow to a breathing circuit, and a drainage port disposed on a lower end portion of the housing. A float valve seat is disposed about said drainage port. The housing further defines a first interior space fluidly coupled to all said ports. A buoyant float body is disposed in the first interior space defining a lower end configured to mate with the float valve seat to seal said drainage port. The buoyant float body is moveable upwards within the interior space when a sufficient amount of liquid accumulates in the interior space to separate the lower end of said buoyant float body from the float valve seat and permit liquid to flow through the drainage port. The buoyant float body defines a volume which displaces at least half of a volume defined by the first interior space defined by the housing exclusive of any volume spanned by the entry and exit ports. And a lower end portion of the device is configured to be coupled to a connector for fluidly coupling the drainage port to a source of suction or negative pressure relative to the first interior space. In a particular embodiment, the lower end portion of the device further defines at least one bleed port for fluidly coupling the source of suction or negative (gauge) pressure to the surroundings outside the device.

In another aspect of the present invention, a method of automatically removing water or accumulated moisture from a breathing circuit includes: providing a housing defining an entry port for receiving flow from a breathing circuit, an exit port for transmitting flow to a breathing circuit, a drainage port disposed on a lower end portion of the housing, and a float valve seat disposed about said drainage port, the housing further defining a first interior space fluidly coupled to all said ports. A buoyant float body is disposed in the first interior space defining a lower end configured to mate with the float valve seat to seal said drainage port, the buoyant float body being moveable upwards within the interior space when a sufficient amount of liquid accumulates in the interior space to separate the lower end of said buoyant float body from the float valve seat and permit liquid to flow through the drainage port, wherein the buoyant float body defines a volume which displaces at least half of a volume defined by the first interior space defined by the housing exclusive of any volume spanned by the entry and exit ports. A container is coupled to the housing for receiving flow exiting from the drainage port, the container defining a second interior space for collection of water or accumulated moisture, the second interior space being closed to the surroundings outside the device. The container further includes a wall bounding said second interior space, said wall made at least in part from a material permeable to water vapor and impermeable to liquid water. The entry and exit ports are coupled to breathing circuit tubing. Water or moisture accumulated in the second interior space is allowed to diffuse through the wall to the surroundings outside the device.

In yet another aspect of the present invention, a method of automatically removing water or accumulated moisture from a breathing circuit includes providing a housing defining an entry port for receiving flow from a breathing circuit, an exit port for transmitting flow to a breathing circuit, and a drainage port disposed on a lower end portion of the housing. A float valve seat is disposed about said drainage port. The housing further defines a first interior space fluidly coupled to all said ports. A buoyant float body is disposed in the first interior space defining a lower end configured to mate with the float valve seat to seal said drainage port. The buoyant float body is moveable upwards within the interior space when a sufficient amount of liquid accumulates in the interior space to separate the lower end of said buoyant float body from the float valve seat and permit liquid to flow through the drainage port. The buoyant float body defines a volume which displaces at least half of a volume defined by the first interior space defined by the housing exclusive of any volume spanned by the entry and exit ports. The entry and exit ports are coupled to breathing circuit tubing. A lower end portion of the device and the drainage port is coupled to a source of suction or negative pressure relative to the first interior space.

There has thus been outlined, rather broadly, certain embodiments of the invention in order that the detailed description thereof herein may be better understood, and in order that the present contribution to the art may be better appreciated. There are additional embodiments of the invention that will be described below and which form the subject matter of the claims appended hereto.

In this respect, before explaining at least one embodiment of the invention in detail, it is to be understood that the invention is not limited in its application to the details of construction and to the arrangements of the components set forth in the following description or illustrated in the drawings. The invention is capable of embodiments in addition to those described and of being practiced and carried out in various ways. Also, it is to be understood that the phraseology and terminology employed herein, as well as the abstract, are for the purpose of description and should not be regarded as limiting.

As such, those skilled in the art will appreciate that the conception upon which this disclosure is based may readily be utilized as a basis for the designing of other structures, methods and systems for carrying out the several purposes of the present invention. It is important, therefore, that the claims be regarded as including such equivalent constructions insofar as they do not depart from the spirit and scope of the present invention.

### BRIEF DESCRIPTION OF THE DRAWINGS

FIG. 1A is a view illustrating a first embodiment of the present invention in assembled form;

FIG. 1B is an exploded view of the embodiment in FIG. 1A;

FIG. 1C is a cross sectional view illustrating the embodiment of FIGS. 1A-1B with the float valve closed;

FIG. 1D is a cross sectional view illustrating the embodiment of FIGS. 1A-1B with the float valve open;

FIG. 2A is a view illustrating another embodiment of the present invention in assembled form;

FIG. 2B is an exploded view of the embodiment in FIG. 2A;

FIG. 2C is a cross sectional view illustrating the embodiment of FIGS. 2A-2B with the float valve closed;

FIG. 2D is a cross sectional view illustrating the embodiment of FIGS. 2A-2B with the float valve open;

FIG. 3A is a view illustrating another embodiment of the present invention in assembled form;

FIG. 3B is an exploded view of the embodiment in FIG. 3A;

FIG. 3C is a cross sectional view illustrating the embodiment of FIGS. 3A-3B with the float valve closed;

FIG. 3D is a cross sectional view illustrating the embodiment of FIGS. 3A-3B with the float valve open;

FIG. 3E is an enlarged cross sectional view illustrating a portion of the embodiment of FIGS. 3A-3B in the position with the float valve closed;

FIG. 3F is an enlarged cross sectional view illustrating a portion of the embodiment of FIGS. 3A-5B in the position with the float valve open;

### DETAILED DESCRIPTION

The invention will now be described with reference to the drawing figures, in which like parts are referred to with like reference numerals throughout. Embodiments in accordance with the present invention provide a water removal or dissipation device and method to remove water vapor or liquid condensate from a humidified medical gas traveling through a breathing circuit between a ventilator and a patient or between a humidification unit and a patient. One or more embodiments provide a means by which the feature or combination of features that removes and/or suctions water from the system is automatic. Traditional water traps require the user to physically drain excess condensation, which requires periodic observation and clinician vigilance. The present invention provides a mechanism to automatically drain accumulated water or excess condensation into a suction source, or a permeable membrane container or other collection reservoir, thus eliminating the need for the clinician to continually monitor and drain the water collection device. The present invention provides a means that removes water or liquid moisture which is isolated from the breathing circuit flow.

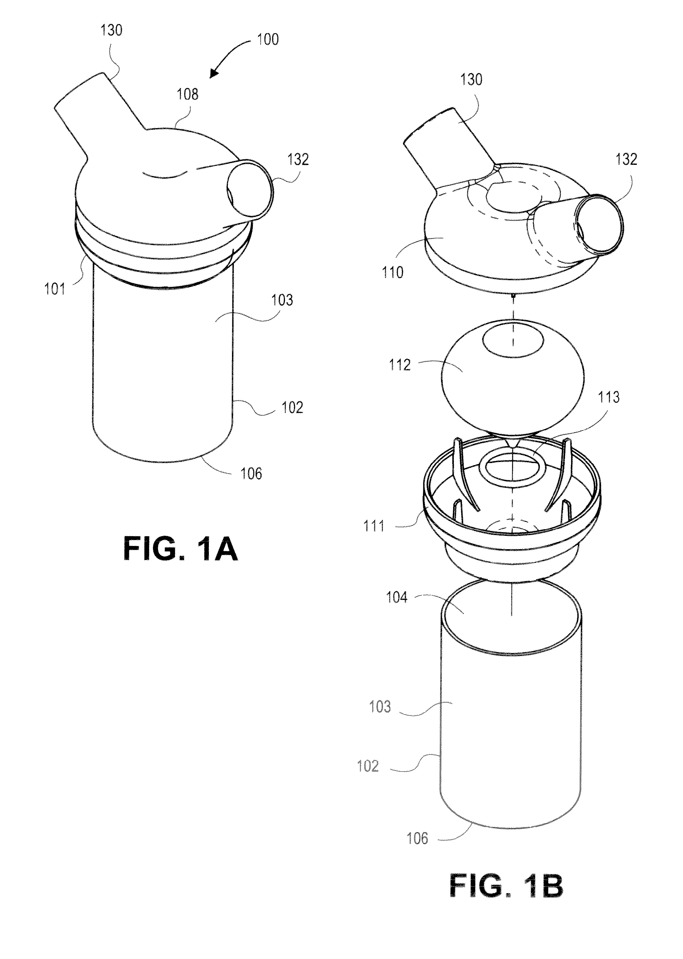

A first embodiment of the present invention is illustrated in FIGS. 1A-1D. FIG. 1B is an exploded view illustrating the water removal or dissipation device 100 which includes a housing 101 coupled to a cylindrical bottom container 102. The cylindrical bottom container 102 has a side wall 103 that defines a top opening 104 and a bottom surface 106. The housing 101 defines an upper portion assembly 108 of the device 100 which is mounted over the top opening 104 of container 102. The housing 101 defines an entry port 130 and an exit port 132. The entry port 130 and the exit port 132 allow the water removal or dissipation device 100 to be connected to a breathing circuit. In the embodiment shown in FIGS. 1A-1D, as best shown in FIG. 1B, the housing 101 includes two parts, an upper 110 and lower part 111 that are attached together to form an interior space in which a buoyant float body 112 is disposed, wherein the float body 112 defines a conical lower end portion around which a circular o-ring 113 is disposed, to form a first part of a float valve mechanism within the device 100.

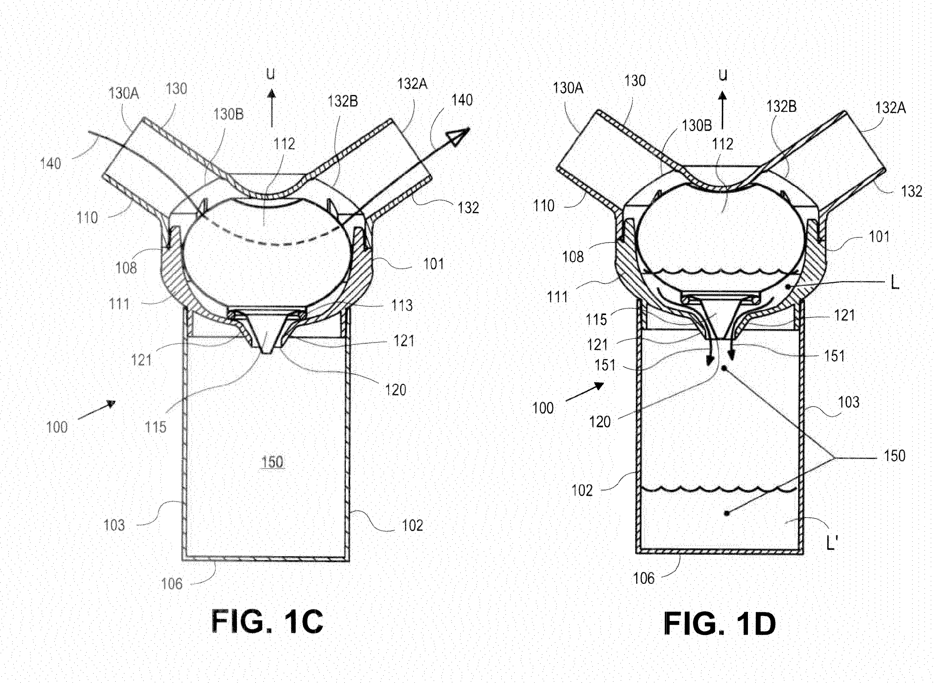

FIGS. 1C-1D are cross sectional views that further illustrate the embodiment of the water removal and dissipation device illustrated in FIGS. 1A-1B. When coupled to a breathing circuit tubing (not shown), the housing 101 defines a flow path 140 of humidified gas between the entry port 130 and the exit port 132. In the flow path 140, the humidified gas travels into the water removal and dissipation device 100 via the entry port 130, through an interior space of the housing 101 and exits the water removal or dissipation device 100 via the exit port 132. The first flow path 140 is therefore indicative of the main breathing flow path through the water removal or dissipation device 100 along the breathing circuit. As shown in FIGS. 1C-1D, a drainage port 120 is disposed on a lower end portion of the housing 101, and a float valve seat 121 is disposed about said drainage port 120. The housing 101 therefore defines an interior space that is fluidly coupled to all of the entry port 130, exit port 140, and drainage port 120.

In the embodiment shown in FIGS. 1A-1D, the buoyant float body 112, which is disposed in the interior space of housing 101, has a generally spheroid-like shape and further defines a projecting, conically-shaped lower end 115 which is configured to mate with the float valve seat 121 to seal said drainage port 120. As shown in FIG. 1D, the buoyant float body 112 is moveable upwards in the direction "U" within the interior space of housing 101 when a sufficient amount of liquid "L" accumulates in the interior space to separate the lower end 115 of said buoyant float body 112 from the float valve seat 121 and thereby permit liquid to flow through the drainage port 120. Furthermore, the buoyant float body 112 defines and occupies a volume which displaces at least half of an inner structural volume defined by the interior space of the housing 101, such space being defined by the structure of the housing parts 110 and 111 coupled together, exclusive of any volume spanned by the entry and exit ports 130 and 132, which ports are delimited in the exemplary embodiment of housing 101 shown in FIGS. 1C-1D by the lines 130A and 130B for entry port 130 and lines 132A and 132B for exit port 132. The amount of volume of interior space within housing 101 displaced by the volume of buoyant float body 112 is at least half of said inner structural volume of housing 101 exclusive of any volume spanned by the entry and exit ports 130, 132, thereby resulting in a "compressible volume" for fluid flow within housing 101 which is significantly reduced. Thus, the present invention provides a means for reducing the compressible volume of the water removal device 100 by a significant amount beyond what is known from the prior art, such as U.S. patent 4,867,153, since the inner buoyant float body 112 occupies a significant and substantial portion of the inner volume spanned by the device which is exposed to the breathing circuit flow, wherein, as explained further below, such inner compressible volume is limited, by virtue of the float valve means therein, to the volume within housing 101 only, and does not include any other space or volume gravitationally beyond drainage port 120, such as collection reservoir 150 in the embodiment shown in FIGS. 1C-1D. This is because as water or liquid condensate accumulates inside the volume within housing 101, shown as "L" in FIG. 1D, the buoyant float body 112 moves upwards within the interior space of housing 101, in the direction "U" as shown in FIGS. 1C-1D. The device 100 is accordingly preferably oriented relative to direction "U" such that said direction is opposite to the pull of gravity, wherein the housing 101 defines an upper portion of device 100, and the container 102 defines a lower portion, relative to gravity. As such, drainage port 120 is disposed on a lower end portion of the housing 101, and provides a gravitationally lowest point whereby water "L" can drain from the port 120 when a sufficient amount of liquid "L" accumulates in the interior space of housing 101 to separate the lower end 115 of said buoyant float body 112 from the float valve seat 121 and permit liquid "L" to flow through the drainage port 120 into container space 150 below it.

As best shown in FIGS. 1C-1D, container 102 is coupled to the housing 101 for receiving flow 151 exiting from the drainage port 120 and to collect such flow as liquid L' at the bottom of said container 102. The container 102 can be sealingly coupled to housing 101, and in one embodiment can also be removable therefrom to enable final disposal of liquid L'. The container 102 thus defines a second interior space 150 for collection of water or accumulated liquid or moisture, the second interior space 150 being closed to the surroundings outside the device 101. In the embodiment shown in FIGS. 1A-1D, the container 102 can be fully impermeable to liquid or gas, such that the present invention provides a means for reducing the compressible volume of the water removal device 100, while also providing a water or liquid collection reservoir 150 that is isolated from the breathing circuit flow 140 within housing 101, since at all times such flow 140 will be sealed from reservoir 150 by action of either: (i) the buoyant float body 112 being sealed to float valve seat 121 to seal drainage port 120, or (ii) the presence of accumulated liquid "L" providing a "water seal" when the float body 112 moves upwards under buoyancy to separate from float valve seat 121 as shown in FIG. 1D. Thus, if leaks form in container 102, the flow path 140 is still isolated from the surroundings.

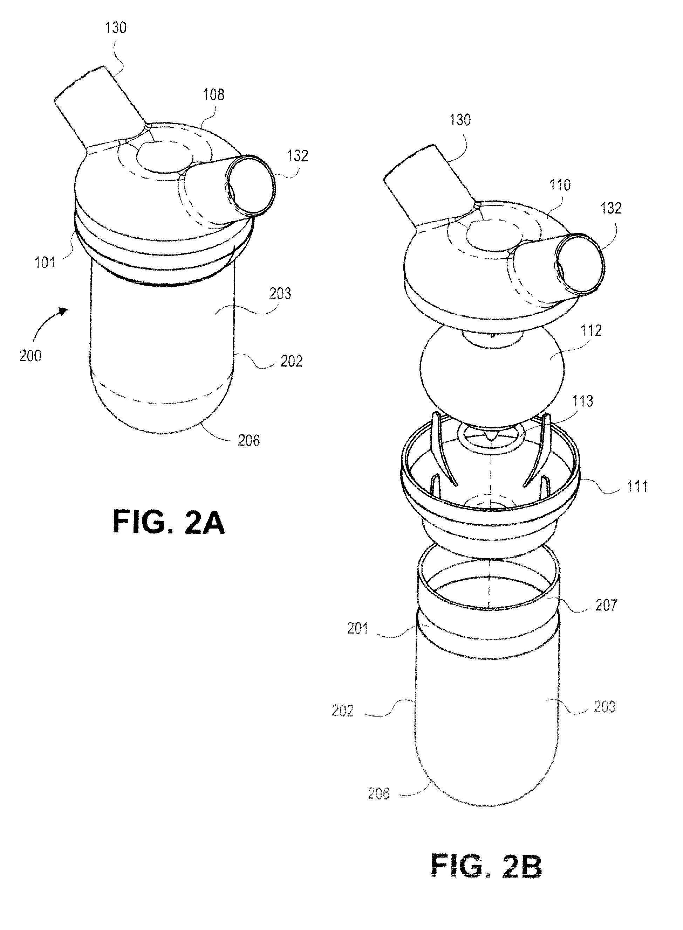

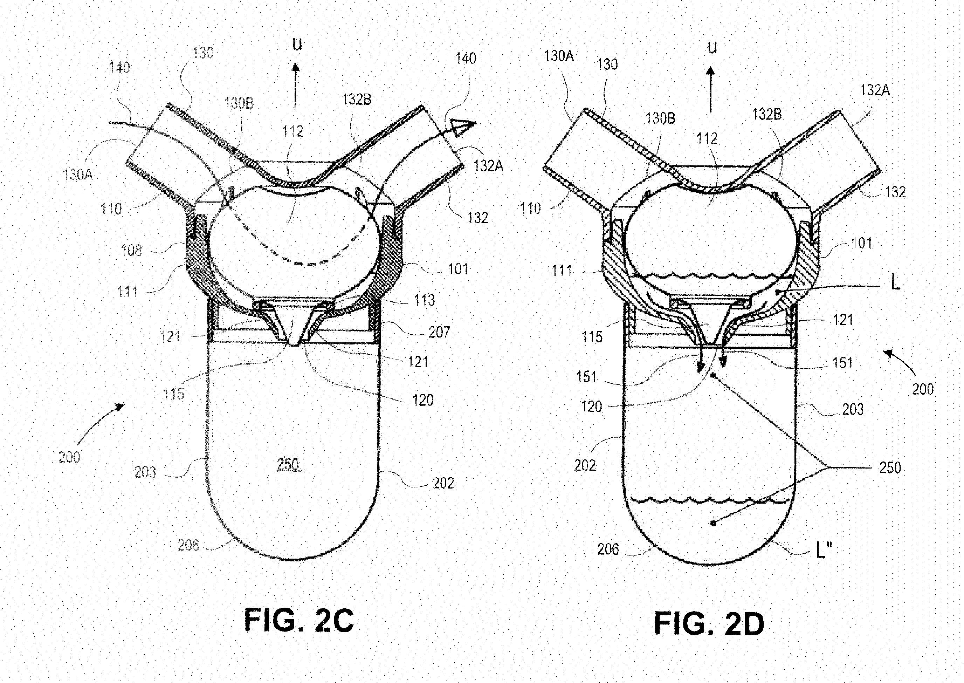

Alternatively, in the embodiment shown in FIGS. 1A-1D, the container 102 may include a wall 103 bounding said second interior space 150, wherein said wall 103 is made at least in part from a material permeable to water vapor and impermeable to liquid water. In such an embodiment, the liquid L' will, over time, automatically dissipate by virtue of osmosis from space 150 to the surroundings outside device 100, due to the water vapor permeability and liquid water impermeability of portions of wall 103, which may further include an inner wicking layer, and/or outer mesh layer for structural rigidity and strength, similar to the devices described and disclosed in commonly owned pending U.S. patent application No. 12/539,088, the disclosure of which is incorporated by reference herein in its entirety. FIGS. 2A-2D are views illustrating another, preferred embodiment of the present invention in assembled form. In FIGS. 2A-2D, an automatic water removal or dissipation device 200 is shown, being similar to device 100, wherein the lower container 202 can be entirely permeable to water vapor and impermeable to liquid water, and is substantially bucket-shaped having a rounded and closed bottom end 206 and an open top end 201 coupled to the housing 101 via an intermediate coupling ring 207. The walls 203 of container 202 are made of material that is permeable to water vapor and impermeable to liquid water, and can be a single or composite material, including a part or layer made of Sympatex® brand water vapor permeable material or membrane made of polymers made by Sympatex Technologies, GmbH. The wall or walls 203 may also be made of a composite of layers of material, also including a wicking layer which can be a knit or non-woven cloth or fabric, made of polyester, polyester and polypropylene blends, nylon, polyethylene or paper, and can be microfilaments or microfiber material such as Evolon® brand fabric material made by Freudenberg & Co. KG. A particular example of wicking material would be a non-woven material of 70% polypropylene and 30% polyester. The wicking layer would promote absorption by wall 203 of the liquid L" accumulated in reservoir 250 such that it may more efficiently and quickly dissipate through to the surroundings outside device 200, as further explained in pending U.S. patent application No. 12/539,088.

In both of the embodiments shown in FIGS. 1A-1D and 2A-2D, the second interior space 150 and 250 is substantially larger in volume than the first interior space and compressible volume within housing 101, as explained above. Indeed the spaces 150 and 250 can be made much larger than any compressible volume within the devices 100, 200, respectively, to provide a means to collect liquid water which is isolated from the breathing circuit flow 140 and the rest of the breathing circuit. In the preferred embodiment shown in FIGS. 2A-2D, the volume provided by inner space 250 spanned by the walls 203 of permeable container 202 can be made significantly larger than prior devices without concern for increasing the compressible volume of the device 200 when coupled to a breathing circuit. The space 250 can be at least 2 to 10 times as large as any inner compressible volume defined by housing 101 as discussed above. Also, in a preferred embodiment, the surface area bounded by walls 203 can be at least 30 to 350 times as large as the cross-sectional area of the breathing circuit flow as measured by the transverse cross-sectional span of entry port 130 when such port represents the total entry of fluid flowing into the device. All of these particular dimensions and shapes enable the relatively large inner flow space and volume 250, and surface area bounded thereby, when compared to the flow through the breathing circuit to which the device is connected, to facilitate water removal and dissipation through permeable membrane walls 203, since the larger volume 250 and corresponding surface area of walls 203 provides not only a larger surface area for transmission through the permeable membrane, but also provides a larger flow volume or space which increases dwell time of water vapor or moisture-containing gas within volume 250, such that it is absorbed by any wicking layer within walls 203 and/or is more quickly or efficiently transmitted through the permeable walls 203, and thus provides a better means for dissipation of water from within the device 200 to the surroundings, all without increasing compressible volume or the compliance of the device and/or the breathing circuit system to which it is coupled.

The float valve mechanism within housing 101 further provides that the device of the present invention, in several embodiments as described herein, can be coupled to an inspiratory limb of a breathing circuit, since the device will not remove or dissipate water or moisture intended for the patient, which should in case reach the patient through such inspiratory limb, unless an excess is present and condenses to accumulate in the housing 101 as described above.

If the device is instead intended for placement in the expiratory limb or intended to remove moisture from the breathing circuit air, the device 100 or 200 can further include means (not shown) for promoting breathing circuit flow cooling and moisture condensation coupled to the entry or exit port 130, 132, such as, for example, a thin wall corrugated tube or other thermally conductive structure arranged on the inside or outside surfaces of the ports 130, 132.

The present invention as disclosed in the embodiments shown in FIGS. 1-2D therefore provides a method of automatically removing water or accumulated moisture from a breathing circuit. The method includes providing a device 100 or 200 having a container 102, 202 which is coupled to a housing 101, which container has a wall made at least in part from a material permeable to water vapor and impermeable to liquid water; or, in a preferred method, entirely permeable to water vapor and impermeable to liquid water. In the method, the device 100 or 200 is coupled via entry and exit ports 130, 132 to breathing circuit tubing (not shown), and water or moisture L' or L" accumulated in the interior space 150 or 250 is allowed to diffuse through the wall 103 or 203 to the surroundings outside the device. The method is automatic in that, once the device 100 or 200 is coupled to the breathing circuit tubing and system, no additional action or user intervention is necessary, as long as water or liquid effectively dissipates through the permeable portions of containers 102 or 202. In the method, the device 100 or 200 can be disposed in an inspiratory limb of a breathing circuit, or, in another particular embodiment, at a junction between heated and unheated portions of the inspiratory limb. Alternatively, in the method, the device 100 or 200 can be disposed in an expiratory limb of a breathing circuit, or, in another particular embodiment, at a junction between heated and unheated portions of the expiratory limb. The method may also include disposing a device 100 or 200 in each of the inspiratory and expiratory limbs, or, in another particular embodiment, at junctions between heated and unheated portions of the inspiratory and expiratory limb. Furthermore, in the method, a means for promoting breathing circuit flow cooling and moisture condensation can be coupled to the entry or exit ports of device 100 or 200.

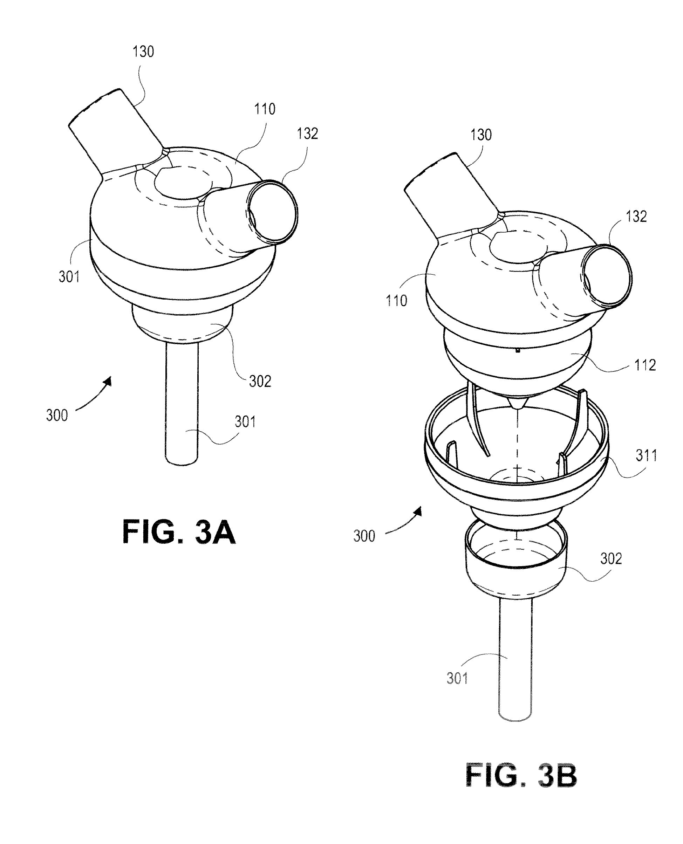

FIGS. 3A-3F are views illustrating another, preferred embodiment of the present invention, including a device 300 having a housing 301 similar to housing 101, with entry port 130 and exit port 132, and made of an upper part 110 and lower part 311 that are attached together to form an interior space in which a buoyant float body 112 is disposed, to form a first part of a float valve mechanism within the device 300, similar to the float valve mechanism in devices 100 and 200 discussed above, and having a drainage port 320 disposed on a lower end portion of the housing 301, with a float valve seat 321 disposed about the drainage port 320. In the device 300, a lower end portion 302 of the device is configured to be coupled to a connector 301 for fluidly coupling a drainage port 320 to a source of suction or negative pressure relative to the interior space within housing 301. The suction or negative (gauge) pressure may be supplied from any suitable means, and such suction or vacuum pressure may be transmitted from the inner lumen 350 of connector tube 301 through device 300 to the inner space defined by housing 301 where liquid "L" may accumulate.

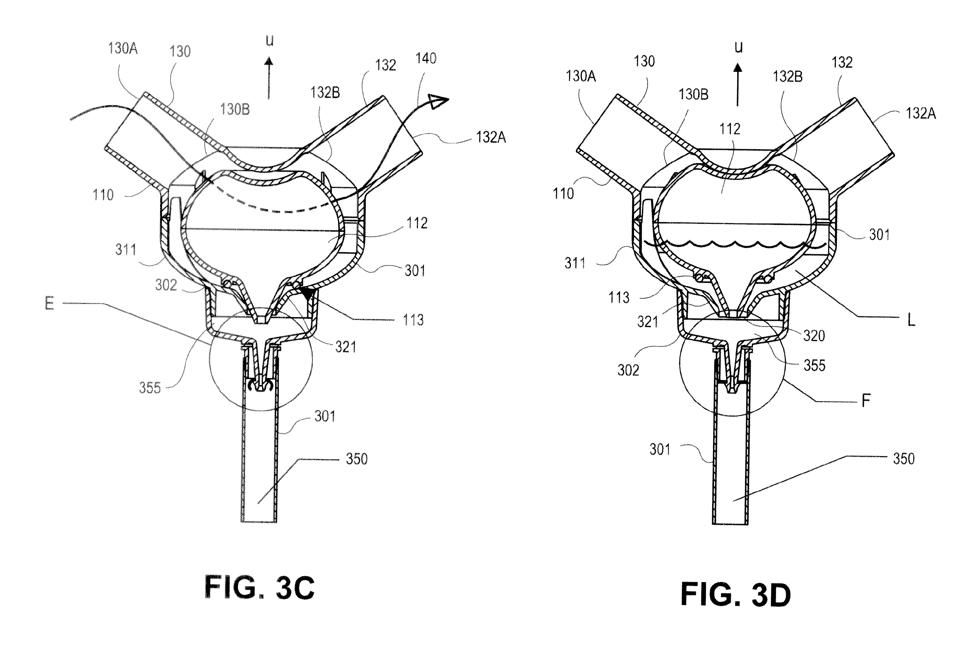

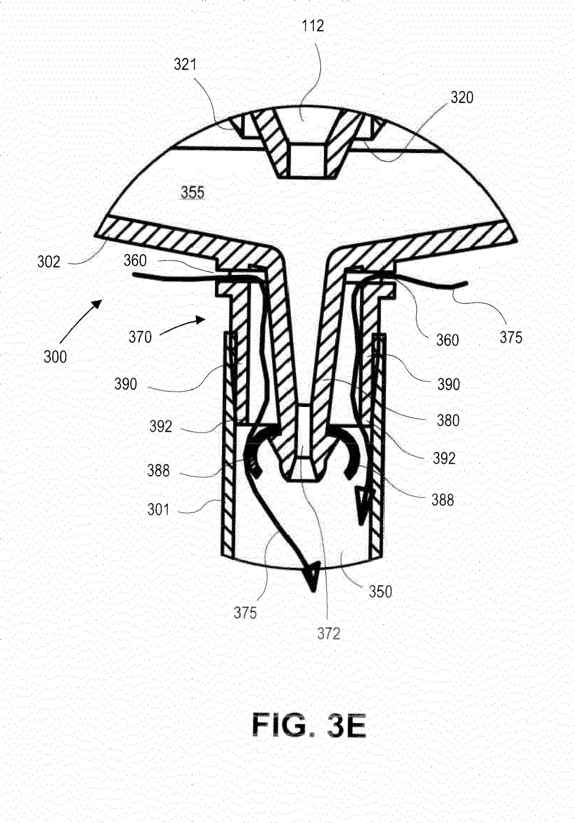

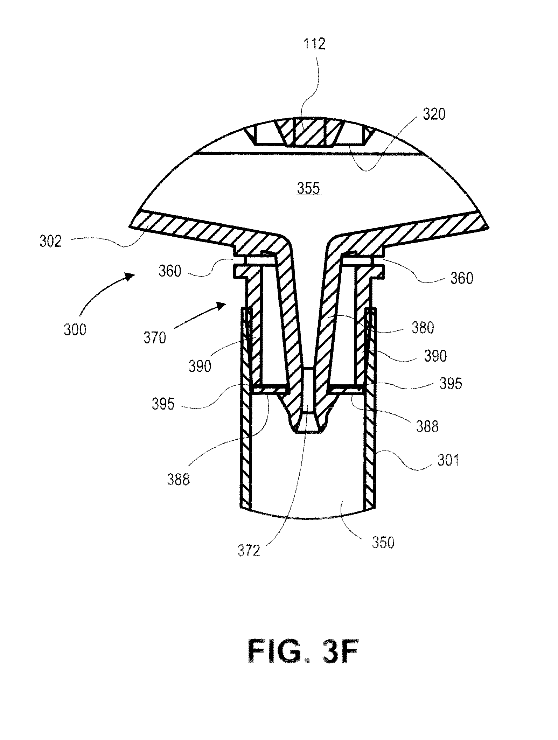

FIG. 3C is a cross sectional view illustrating the embodiment of FIGS. 3A-3B with the float valve mechanism closed. FIG. 3E is an enlarged cross sectional view of area "E" in FIG. 3C. FIG. 3D is a cross sectional view illustrating the embodiment of FIGS. 3A-3B with the float valve mechanism open. FIG. 3F is an enlarged cross sectional view of area "F" in FIG. 3D. As best shown in FIG. 3F, a lower end portion of the device 300 further defines at least one bleed port 360 for fluidly coupling the source of suction or negative pressure via connector lumen 350 to the surroundings outside the device. Furthermore, the lower end portion 302 of the device 300 may include a drainage flow receiving section 370 which defines a lower opening 372 to permit drainage of liquid therethough. In the embodiment shown in FIGS. 3E-3F, said drainage flow receiving section 370 defines two bleed ports 360. The bleed ports 360 allow pressure to equalize between the surroundings, at ambient or atmospheric pressure outside device 300, and the negative or vacuum pressure applied by the source of suction to connector lumen 350, which negative pressure is transmitted through opening 372 to drainage port 320 of housing 301 and thereby into the interior space inside the housing 301. When no water or liquid is present in such space, the buoyant float body 112 is seated on float valve seat 321 as shown in FIG. 3C, sealing the interior space inside housing 301. However the negative pressure transmitted from lumen 350 into a second interior space 355 enclosed by the lower end portion 302 of device 300 coupled to housing 301 will in such a position tend to create a positive pressure differential between the interior space of housing 301 and the space 355 outside the housing 301, causing the buoyant float body 112 to be firmly seated onto float valve seat 321. If no bleed ports 360 are present, such a pressure differential may be undesirable in that it may make it difficult to unseat float body 112 and open discharge opening 320 to drain water even when a significant amount of liquid has accumulated inside housing 301. The pressure differential may exceed the counteracting buoyancy forces on float body 112. To mitigate this and reduce the potential pressure differential, the bleed ports 360 are positioned and sized to reduce the suction or vacuum pressure applied and transmitted to space 355 via opening 372, by equalizing the pressure between suction connector lumen 350 and the ambient air, as shown by flow arrows 375 in FIG. 3E. To further accomplish this, the drainage flow receiving section 370 further defines a one-way valve element which is opened to permit flow through the bleed ports 360 to the source of suction or negative pressure when the float body 112 is mated on the float valve seat 321 sealing the drainage port 320 of the housing 301. As shown in FIGS. 3E and 3F, the drainage flow receiving section 370 includes a narrow conical neck 380 defining the lower opening 372, and a tubular element 390 concentrically surrounding said neck 380 and defining a lower end port 392 configured to be inserted into tubular lumen 350 of connector 301. The one-way valve element includes at least one flap 388 extending from the neck 380 to a lower rim 395 of the tubular element 390. In the position shown in FIG. 3F, the flap 388, which may be shaped as a flexible circular ring element, is attached to neck element 380 and closed over tubular element 390 to close off bleed ports 360 and the surroundings from suction lumen 350 and the rest of the interior of device 300. Water or liquid L which has accumulated in the interior space of housing 301 may drain through port 320 when sufficient buoyancy pushes float body 112 away from float valve seat 321 to permit water or liquid to drain through to receiving space 355 and on through opening 372 into the suction connector 301 for complete removal from device 301. Once a sufficient amount of drainage has occurred, the float valve body 112 will be seated back onto float valve seat 321 to close drainage port 320, and continued suction applied through lumen 350 will cause the flap 388 to be pulled downward away from the rim 395 of tubular element 390 so as to permit flow 375 from bleed ports 360 to the suction lumen 350, thereby reducing the suctioning force that may unnecessarily force float body 112 seated onto float valve seat 321. The size of the bleed ports 360 can be calibrated to the amount of suction applied through connector 301 to achieve the desired operation as described herein. The device 300 therefore includes a first liquid drainage port 320 that drains liquid into a first fluid receiving space 355, which narrows downwards into a second liquid drainage port 372 which is fluidly coupled a source of suction via connector lumen 350. The valve element 388 separates the source of suction or lumen 350 from the ambient surroundings outside device 300 by providing a barrier between the source of suction in lumen 350 and the bleed ports 360 defined by a structural portion of device 300, such as for example lower end portion 302, or, in a particular embodiment, the drainage flow receiving section 370 which is coupled to, or integrally made with, housing 301. Thus, a first flow pathway, for liquid drainage, is defined from space 355 through orifice 372 to the suction lumen 350, while a second flow pathway, for pressure equalization, is defined from bleed ports 360, past valve element 388 (when opened as in FIG. 3E) to the suction lumen 350.

If the device 300 is intended for placement in the expiratory limb or intended to remove moisture from the breathing circuit air, the device 300 can further include means (not shown) for promoting breathing circuit flow cooling and moisture condensation coupled to the entry or exit port 130, 132, such as for example, a thin wall corrugated tube or other thermally conductive structure arranged on the inside or outside surfaces of the ports 130, 132.

The present invention as disclosed in the embodiments shown in FIGS. 3A-3F therefore provides a method of automatically removing water or accumulated moisture from a breathing circuit. The method includes providing a device 300 which is coupled to the source of suction or negative pressure (not shown) via connector 301. In the method, the device 300 is coupled via entry and exit ports 130, 132 to breathing circuit tubing (not shown), and water or moisture L' or L" accumulated in the interior space of housing 301 can drain through to the suction source as described above. The method is automatic in that, once the device 300 is coupled to the breathing circuit tubing and system, no additional action or user intervention is necessary, as long as suction is applied, and the float valve mechanism works as described above in cooperation with the bleed ports 360 so as not to cause float body 112 to be seated too firmly against float valve seat 321, thereby allowing proper periodic separation from the float valve seat 321 to permit drainage of water when a sufficient amount has accumulated in housing 301. In the method, the device 300 can be disposed in an inspiratory limb of a breathing circuit, or, in another particular embodiment, at a junction between heated and unheated portions of the inspiratory limb. Alternatively, in the method, the device 300 can be disposed in an expiratory limb of a breathing circuit, or, in another particular embodiment, at a junction between heated and unheated portions of the expiratory limb. The method may also include disposing a device 300 in each of the inspiratory and expiratory limbs, or, in another particular embodiment, at junctions between heated and unheated portions of the inspiratory and expiratory limb. Furthermore, in the method, a means for promoting breathing circuit flow cooling and moisture condensation can be coupled to the entry or exit ports of device 300.

The invention therefore provides a device and method that reduces and minimizes the compressible volume within any breathing circuit system to which the device is coupled, while providing a constant water barrier to isolate the breathing circuit flow from the surroundings while accumulated water and/or water vapor is effectively removed or dissipated from the system. This allows for placement of the water removal or dissipation device in the inspiratory limb of a breathing circuit because the device will not remove moisture intended for the patient. As described herein, the present invention significantly reduces the compliance and compressible volume of previously known water dissipation devices, and reduces the criticality of potential pin hole leaks in similar devices using permeable membranes as the flow within the circuit is isolated from the membrane, thereby eliminating a direct air leak path, and further eliminates the concern of aerosol treatments within the breathing circuit flow affecting such permeable membrane embodiments, and allows for larger permeable membrane embodiments without increasing breathing circuit compressible volume and thus helping in increasing the water transmission rate.

The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, all suitable modifications and equivalents may be resorted to, falling within the scope of the invention.

## Claims

1. A device for automatically removing water or accumulated moisture from a breathing circuit, comprising:
a housing defining
an entry port for receiving flow from the breathing circuit,
an exit port for transmitting flow to the breathing circuit,
a drainage port disposed on a lower end portion of the housing, and a float valve seat disposed about said drainage port, the housing further defining a first interior space fluidly coupled to all said ports,
a buoyant float body disposed in the first interior space defining a lower end configured to mate with the float valve seat to seal said drainage port, the buoyant float body being moveable upwards within the interior space when a sufficient amount of liquid accumulates in the interior space to separate the lower end of said buoyant float body from the float valve seat and permit liquid to flow through the drainage port, wherein the buoyant float body defines a volume which displaces at least half of a volume defined by the first interior space defined by the housing exclusive of any volume spanned by the entry and exit ports, and
a container coupled to the housing for receiving flow exiting from the drainage port, the container defining a second interior space for collection of water or accumulated moisture, the second interior space being closed to the surroundings outside the device, and the container further comprising a wall bounding said second interior space, said wall made at least in part from a material permeable to water vapor and impermeable to liquid water.

2. The device of claim 1, wherein the entirety of said container wall is permeable to water vapor and impermeable to liquid water.

3. The device of claim 2, wherein the container is substantially bucket-shaped having a closed bottom end and an open top end coupled to the housing.

4. The device of claim 1, wherein the second interior space is substantially larger in volume than the first interior space.

5. A device for automatically removing water or accumulated moisture from a breathing circuit, comprising:
a housing defining
an entry port for receiving flow from the breathing circuit,
an exit port for transmitting flow to the breathing circuit, and
a drainage port disposed on a lower end portion of the housing, and a float valve seat disposed about said drainage port, the housing further defining a first interior space fluidly coupled to all said ports,
a buoyant float body disposed in the first interior space defining a lower end configured to mate with the float valve seat to seal said drainage port, the buoyant float body being moveable upwards within the interior space when a sufficient amount of liquid accumulates in the interior space to separate the lower end of said buoyant float body from the float valve seat and permit liquid to flow through the drainage port, wherein the buoyant float body defines a volume which displaces at least half of a volume defined by the first interior space defined by the housing exclusive of any volume spanned by the entry and exit ports, and wherein a lower end portion of the device is configured to be coupled to a connector for fluidly coupling the drainage port to a source of suction or negative pressure relative to the first interior space.

6. The device of claim 5, wherein the lower end portion of the device further defines at least one bleed port for fluidly coupling the source of suction or negative pressure to the surroundings outside the device.

7. The device of claim 6, wherein the lower end portion of the device comprises a drainage flow receiving section which defines a lower opening to permit drainage of liquid therethough, the drainage flow receiving section defining the at least one bleed port.

8. The device of claim 7, the drainage flow receiving section further defining a one-way valve element, said valve element being opened to permit flow through the at least one bleed port to the source of suction or negative pressure when the float body is mated on the float valve seat sealing the drainage port of the housing.

9. The device of claim 8, the drainage flow receiving section further comprising a conical neck defining the lower opening, and a tubular element surrounding said neck and defining a lower end port configured to be inserted into a tubular lumen of said connector, and the one-way valve element comprises at least one flap extending from the neck to a rim of the tubular element.

10. The device of claim 1 or claim 5, further comprising means for promoting breathing circuit flow cooling and moisture condensation coupled to the entry or exit port.

11. A method of automatically removing water or accumulated moisture from a breathing circuit, comprising the steps of:
providing a device as claimed in one of claims 1, 2, 3, 4, or 10,
coupling the entry and exit ports to breathing circuit tubing, and
allowing water or moisture accumulated in the second interior space to diffuse through the wall to the surroundings outside the device.

12. A method of automatically removing water or accumulated moisture from a breathing circuit, comprising the steps of:
providing a device as claimed in one of claims 5, 6, 7, 8, 9, or 10,
coupling the entry and exit ports to breathing circuit tubing,
coupling a lower end portion of the device and the drainage port to a source of suction or negative pressure relative to the first interior space.

13. The method of claim 11 or 12, wherein the breathing circuit tubing is an inspiratory limb of said breathing circuit.

14. The method of claim 13, wherein the housing is disposed at a junction between heated and unheated portions of the inspiratory limb.

15. The method of claim 11 or 12, wherein the breathing circuit tubing is an expiratory limb of said breathing circuit.

16. The method of claim 15, wherein the housing is disposed at a junction between heated and unheated portions of the expiratory limb.
